# EUROPEAN PATENT APPLICATION

(11) **EP 3 903 868 A1**
(43) Date of publication of application: **03.11.2021**
(21) Application number: 21171823.4
(22) Date of filing: 03.05.2021
(51) Int. Cl.: A61M 16/06

(54) **CPAP MASK WITH SIDE PORTS FOR PROVIDING CONTINUOUS POSITIVELY PRESSURIZED AIR TO A PATIENT WITH RESPIRATORY DISTRESS AND OTHER RESPIRATORY MEDICAL CONDITIONS**

(30) Priority: 01.05.2020 IN 202011018640
(71) Applicant: Sterlite Technologies Limited, Haryana 122002 (IN)
(72) Inventor: ASTHANA, Sonal, 122002 Haryana (IN); AGGARWAL, Ankit, 122002 Haryana (IN); KUMAR, Shantha, 122002 Haryana (IN)
(74) Representative: Hepworth Browne

(57) **Abstract**

The present disclosure provides a CPAP mask (102) with side ports for providing continuous positively pressurized air to a patient (124). The CPAP mask (102) includes a mask body, a plurality of strap holders (114), and a cushion seal (116). The mask body includes a left side wall (104), a left port (106), a front face (110), a right side wall (112), and a right port (108). In addition, the left side wall (104) has a first through-hole. Further, the left port (106) is on the left side wall (104). Furthermore, the front face (110) of the CPAP mask (102) is a transparent member. Moreover, the right side wall (112) has a second through-hole. Also, the right port (108) is on the right side wall (112). Also, the plurality of strap holders (114) is on the left side wall (104) and the right side wall (112).

## Description

### TECHNICAL FIELD

The present disclosure relates to the field of respiratory devices and, in particular, relates to a Continuous Positive Airway Pressure (CPAP) mask with side ports for providing continuous positively pressurized air to a patient with respiratory distress or respiratory medical conditions.

### BACKGROUND

Respiratory disorders are potentially life-threatening disorders characterized by brief interruptions of breathing. The respiratory disorders include asthma, chronic obstructive pulmonary disease (COPD), pulmonary fibrosis, sleep apnea, pneumonia, lung cancer, and the like. In addition, early recognition and treatment of the respiratory disorders is important. Further, positive airway pressure is an effective form of therapy for a patient suffering from a respiratory disorder where there is inflammation of the lung internal walls caused by COVID19 virus, SARS virus, MERS, Influenza virus and the like. Recently, it has been established by the International Medical Community that a Non-invasive form of respiratory support is most ideal and best treatment procedure to treat patients on a very large scale that the world is faced with due to COVID19 Pandemic. In addition, CPAP is the Non-invasive treatment and is now widely adopted in treating Patients affected with COVID19 Virus. Further, ventilation is applied in form of continuous positive airway pressure (CPAP), in which positive pressure is maintained in the airway throughout the respiratory cycle. Furthermore, the patient wears a mask over mouth and/or nose for continuous positive airway pressure (CPAP). However, current masks apply excessive pressure to the patient's face resulting in discomfort and possibly skin irritation. In addition, the current marks are inefficient to allow outflow of excessive carbon dioxide exhaled by the patient. Further, the current masks have ports and other devices on front face of respiratory face mask. Furthermore, the other devices include tubes, PEEP valves, filters, and the like. Moreover, the current masks with front ports cannot facilitate treatment of the patient lying down in prone or face down position due to obstruction caused by the front ports of the respiratory face mask and the other devices.

In light of the above-stated discussion, there is a need to overcome the above stated disadvantages.

### OBJECT OF THE DISCLOSURE

A primary object of the present disclosure is to provide a CPAP mask to provide continuous positively pressurized air to a patient.

Another objective of the present disclosure is to provide the CPAP mask to minimize pressure on face of the patient to provide comfort fitting.

Yet another object of the present disclosure is to provide the CPAP mask to allow outflow of excessive carbon dioxide exhaled by the patient.

Yet another object of the present disclosure is to provide the CPAP mask to enable a doctor to view features of the patient through transparent front face.

Yet another object of the present disclosure is to provide the CPAP mask with side ports to facilitate treatment of the patient lying in prone position or face down position

### SUMMARY

In an aspect, the present disclosure provides the CPAP mask. The CPAP mask with side ports for providing continuous positively pressurized air to a patient. The CPAP mask includes a mask body, a plurality of strap holders, and a cushion seal. The mask body includes a left side wall, a left port, a front face, a right side wall, and a right port. In addition, the left side wall has a first through-hole. Further, the left port is on the left side wall. Furthermore, the front face of the CPAP mask is a transparent member. Moreover, the front face allows a plurality of medical practitioners to view one or more facial features of the patient. Also, the right side wall has a second through-hole. Also, the right port is on the right side wall. Also, the plurality of strap holders is on the left side wall and the right side wall of the mask body. Also, the plurality of strap holders clamps a medical mask strap. Also, the medical mask strap holds head of the patient with the CPAP mask. Also, the cushion seal is fastened along a perimeter of the CPAP mask. Also, the cushion seal connects the CPAP mask to facial area of the patient.

In an embodiment of the present disclosure, the CPAP mask securely covers nose and mouth of the patient.

In an embodiment of the present disclosure, the right port is connected with a ventilator through a flexible hose. In addition, the flexible hose allows continuous positive airway pressure ventilation for the patient. Further, the patient receives oxygenated air from the ventilator through the flexible hose.

In an embodiment of the present disclosure, the left port is aligned with the right port. In addition, the alignment enables unobstructed flow of the oxygenated air from the ventilator.

In an embodiment of the present disclosure, the cushion seal flexes according to shape of the facial area of the patient. In addition, the cushion seal seals the CPAP mask with the facial area of the patient.

In an embodiment of the present disclosure, the left port of the CPAP mask is connected to a PEEP valve and a HEPA filter unit. In addition, the PEEP valve maintains pressure of expiration air of the patient. Further, the HEPA filter unit traps a plurality of bacteria and viruses and a plurality of particulates. Furthermore, the HEPA filter unit filters outflow of the expiration air of the patient.

In an embodiment of the present disclosure, the CPAP mask is utilized for a plurality of positions. In addition, the patient rests in the plurality of positions. Further, the plurality of positions includes supine position and prone position.

### STATEMENT OF THE DISCLOSURE

The present disclosure talks about the CPAP mask. The CPAP mask with side ports for providing continuous positively pressurized air to a patient. The CPAP mask includes a mask body, a plurality of strap holders, and a cushion seal. The mask body includes a left side wall, a left port, a front face, a right side wall, and a right port. In addition, the left side wall has a first through-hole. Further, the left port is on the left side wall. Furthermore, the front face of the CPAP mask is a transparent member. Moreover, the front face allows a plurality of medical practitioners to view one or more facial features of the patient. Also, the right side wall has a second through-hole. Also, the right port is on the right side wall. Also, the plurality of strap holders is on the left side wall and the right side wall of the mask body. Also, the plurality of strap holders clamps a medical mask strap. Also, the medical mask strap holds head of the patient with the CPAP mask. Also, the cushion seal is fastened along a perimeter of the CPAP mask. Also, the cushion seal connects the CPAP mask to facial area of the patient.

### BRIEF DESCRIPTION OF FIGURES

Having thus described the disclosure in general terms, reference will now be made to the accompanying figures, wherein:
**FIG. 1** illustrates a first side view of a CPAP mask, in accordance with various embodiments of the present disclosure;
**FIG. 2** illustrates a front view of the CPAP mask, in accordance with various embodiments of the present disclosure;
**FIG. 3** illustrates a second side view of the CPAP mask, in accordance with various embodiments of the present disclosure;
**FIG. 4** illustrates a third side view of the CPAP mask, in accordance with various embodiments of the present disclosure;
**FIG. 5** illustrates a fourth side view of the CPAP mask with a cushion seal, in accordance with various embodiments of the present disclosure;
**FIG. 6** illustrates a fifth side view of the CPAP mask with the cushion seal, in accordance with various embodiments of the present disclosure;
**FIG. 7** illustrates a front view of a patient with the CPAP mask, a PEEP valve and a HEPA filter unit, in accordance with an embodiment of the present disclosure;
**FIG. 8** illustrates a front view of the CPAP mask with the PEEP valve and the HEPA filter unit, in accordance with another embodiment of the present disclosure;
**FIG. 9** illustrates a first side view of the HEPA filter unit, in accordance with an embodiment of the present disclosure;
**FIG. 10** illustrates a second side view of the HEPA filter unit, in accordance with another embodiment of the present disclosure;
**FIG. 11** illustrates a first side view of the PEEP valve, in accordance with an embodiment of the present disclosure; and
**FIG. 12** illustrates a second side view of the PEEP valve, in accordance with another embodiment of the present disclosure.

It should be noted that the accompanying figures are intended to present illustrations of exemplary embodiments of the present disclosure. These figures are not intended to limit the scope of the present disclosure. It should also be noted that accompanying figures are not necessarily drawn to scale.

### DETAILED DESCRIPTION

In the following description, for purposes of explanation, numerous specific details are set forth in order to provide a thorough understanding of the present technology. It will be apparent, however, to one skilled in the art that the present technology can be practiced without these specific details. In other instances, structures and devices are shown in block diagram form only in order to avoid obscuring the present technology.

Reference in this specification to "one embodiment" or "an embodiment" means that a particular feature, structure, or characteristic described in connection with the embodiment is included in at least one embodiment of the present technology. The appearance of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but no other embodiments.

Moreover, although the following description contains many specifics for the purposes of illustration, anyone skilled in the art will appreciate that many variations and/or alterations to said details are within the scope of the present technology. Similarly, although many of the features of the present technology are described in terms of each other, or in conjunction with each other, one skilled in the art will appreciate that many of these features can be provided independently of other features. Accordingly, this description of the present technology is set forth without any loss of generality to, and without imposing limitations upon, the present technology.

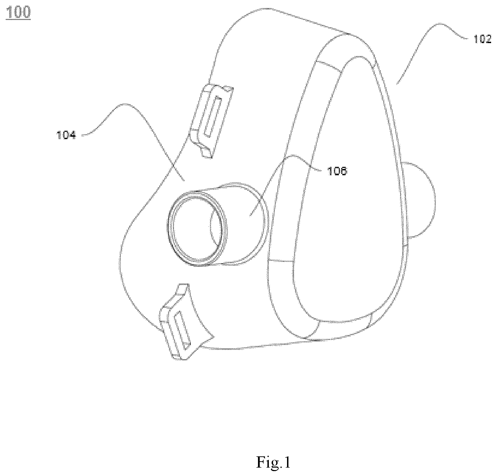

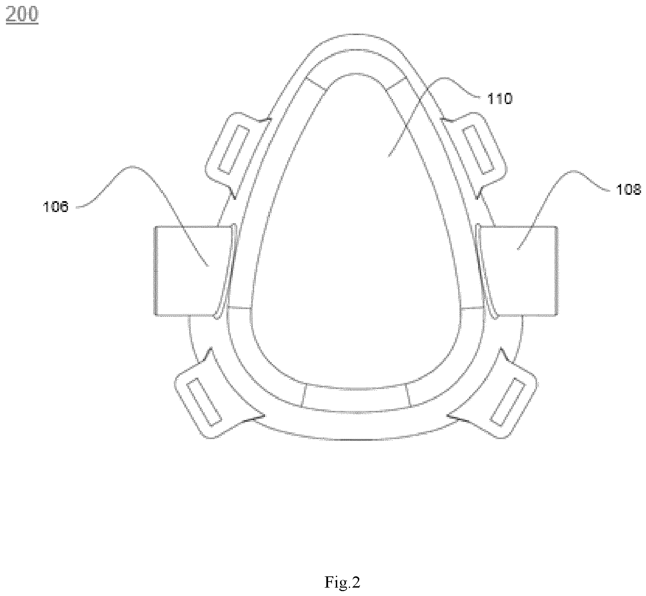

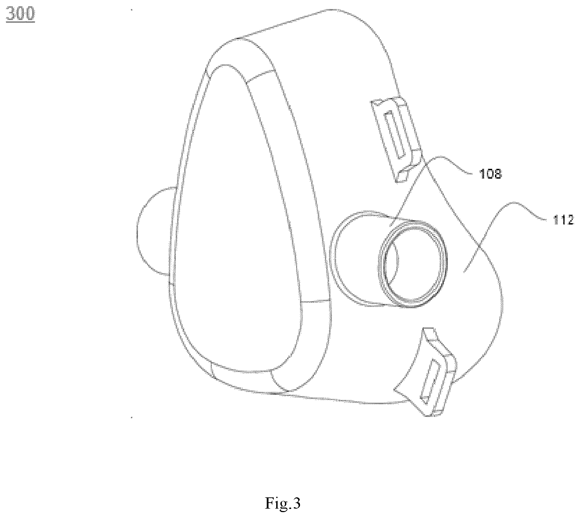

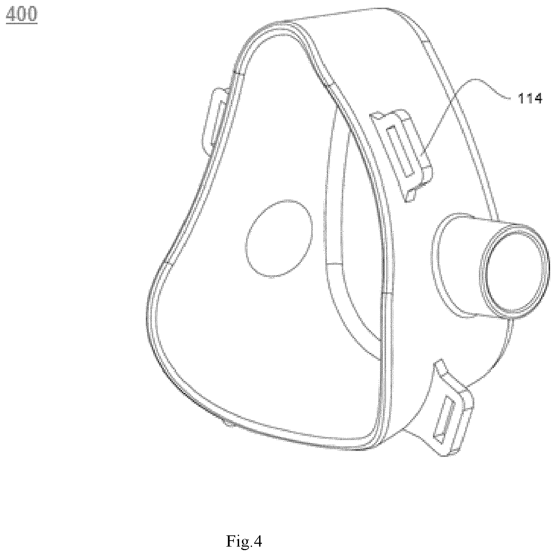

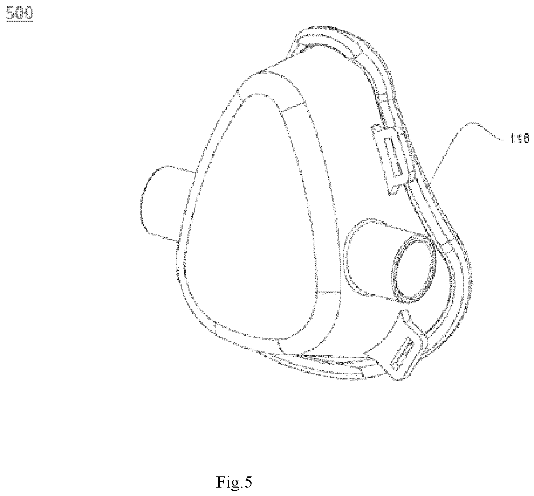

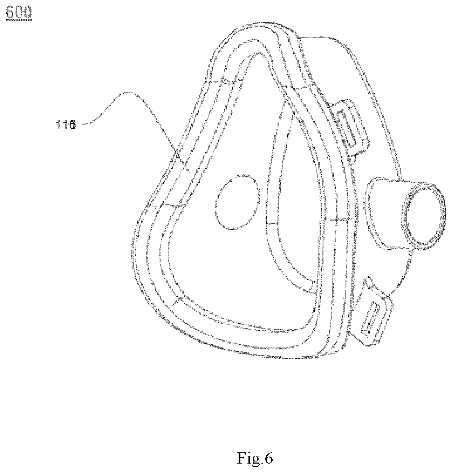

**FIG. 1** illustrates a first side view **100** of the CPAP mask **102,** in accordance with various embodiments of the present disclosure. **FIG. 2** illustrates a front view **200** of the CPAP mask **102,** in accordance with various embodiments of the present disclosure. **FIG. 3** illustrates a second side view **300** of the CPAP mask **102,** in accordance with various embodiments of the present disclosure. **FIG. 4** illustrates a third side view **400** of the CPAP mask **102,** in accordance with various embodiments of the present disclosure. **FIG. 5** illustrates a fourth side view **500** of the CPAP mask **102** with a cushion seal **116,** in accordance with various embodiments of the present disclosure. **FIG. 6** illustrates a fifth side view **600** of the CPAP mask **102** with the cushion seal **116,** in accordance with various embodiments of the present disclosure. In an embodiment of the present disclosure, the CPAP mask **102** is utilized for a plurality of positions. In addition, the patient **124** rests in the plurality of positions. Further, the plurality of positions includes supine position and prone position. In an embodiment of the present disclosure, the patient **124** lies in supine position. In another embodiment of the present disclosure, the patient **124** lies in prone position. The CPAP mask **102** has a cup-shaped mask body when worn to fit over a person's nose and mouth.

The CPAP mask **102** with side ports provides continuous positively pressurized air to a patient **124.** In addition, the CPAP mask **102** corresponds to a continuous positive airway pressure mask. Further, the CPAP mask **102** maintains the continuous positive airway pressure for the patient **124.** In an embodiment of the present disclosure, the patient **124** is suffering from one or more respiratory disorders. In addition, the one or more respiratory disorders include but may not be limited to asthma, chronic obstructive pulmonary disease (COPD), pulmonary fibrosis, sleep apnea, pneumonia, lung cancer, and Coronavirus (COVID-19). In an embodiment of the present disclosure, the patient **124** suffers from a respiratory disorder of the one or more respiratory disorders. In addition, the respiratory disorder of the one or more respiratory disorders causes inflammation on internal walls of lung due to a plurality of viruses. Further, the plurality of viruses include but may not be limited to COVID19 virus, SARS virus, MERS, and Influenza virus. Furthermore, the patient **124** requires a non-invasive form of respiratory support. In an embodiment of the present disclosure, the non-invasive form of respiratory support is CPAP. In addition, CPAP corresponds to continuous positive airway pressure. Further, the CPAP mask **102** securely covers nose and mouth of the patient **124.** In an example, a patient PI is suffering from acute pneumonia due to Corona Virus. In addition, the patient PI is not able to breathe. Further, a CPAP mask M1 connected with a ventilator VI is utilized to assist the patient P1.

The CPAP mask **102** includes a mask body, a plurality of strap holders **114,** and the cushion seal **116.** The mask body includes a left side wall **104,** a left port **106,** a front face **110,** a right side wall **112,** and a right port **108.** The mask body is a cup-shaped body of the CPAP mask **102** that fits on face of the patient **124.** The mask body has hollow space to cover mouth and nose of the patient **124.** In addition, the left side wall **104** has a first through-hole. Further, the first through-hole is provided to facilitate the left port **106.** In an embodiment of the present disclosure, number of the plurality of strap holders **114** on the left side wall **104** is 2. In another embodiment of the present disclosure, number of the plurality of strap holders **114** on the left side wall **104** may vary. In addition, the left port **106** is on the left side wall **104.**

In an embodiment of the present disclosure, the front face **110** of the CPAP mask **102** is a transparent member. In addition, the front face **110** allows a plurality of medical practitioners to view one or more facial features of the patient **124.** Further, the one or more medical practitioners include but may not be limited to doctors, nurses, clinical assistant, patient services assistant, and porter. Furthermore, the front face **110** creates an internal breathing chamber for the patient **124.** In an example, a patient PI might be suffering from Covid-19. In addition, a doctor D1 puts a CPAP mask M1 covering mouth and nose of the patient P1. Further, the doctor D1 views breathing difficulty, opened mouth, and narrower maxillae of the patient PI through transparent member of the CPAP mark M1.

The right side wall **112** has a second through-hole. In addition, the second through-hole is provided to facilitate the right port **108.** In an embodiment of the present disclosure, number of the plurality of strap holders **114** on the right side wall **112** is 2. In another embodiment of the present disclosure, number of the plurality of strap holders **114** on the right side wall **112** may vary. In addition, the right port **108** is on the right side wall **112.** Further, the left port **106** is aligned with the right port **108.** Furthermore, the alignment enables unobstructed flow of oxygenated air from a ventilator. Moreover, the left port **106** and the right port **108** are the side ports of the CPAP mask **102.** In general, ventilator supplies breathable air. In addition, the ventilator delivers the breathable air to patient who is physically unable to breathe.

The plurality of strap holders **114** is on the left side wall **104** and the right side wall **112.** In addition, the plurality of strap holders **114** clamps a medical mask strap. In an embodiment of the present disclosure, the medical mask strap holds head of the patient **124** with the CPAP mask **102.** In another embodiment of the present disclosure, the medical mask strap wraps around ear of the patient **124.** In an embodiment of the present disclosure, the medical mask strap is made up of cloth fabric. In another embodiment of the present disclosure, the medical mask strap may be made up of any other type of fabric. In yet another embodiment of the present disclosure, the medical mask strap is made up of rubber. In yet another embodiment of the present disclosure, the medical mask strap is made up of plastic. In yet another embodiment of the present disclosure, the medical mask strap may be made up of any other material.

The cushion seal **116** is fastened along a perimeter of the CPAP mask **102.** The perimeter is a path that surrounds a two-dimensional shape of the CPAP mask **102** that touches facial area of the patient **124.** The perimeter of the CPAP mask **102** is covered with the cushion seal **116** to provide protection and comfort to the patient **124.** In addition, the cushion seal **116** connects the CPAP mask **102** to facial area of the patient **124.** In an embodiment of the present disclosure, the cushion seal **116** provides cushioning to facial area of the patient **124** in prone position. In another embodiment of the present disclosure, the cushion seal **116** provides cushioning to facial area of the patient **124** in supine position. In addition, the cushion seal **116** flexes according to shape of the facial area of the patient **124.** Further, the cushion seal **116** seals the CPAP mask **102** with the facial area of the patient **124.** In an embodiment of the present disclosure, the cushion seal **116** is made up of a thick medical grade soft rubber. In another embodiment of the present disclosure, the cushion seal **116** is made up of a silicone elastomer. In addition, the cushion seal **116** forms air chamber between the facial area of the patient **124** and the CPAP mask **102.**

The CPAP mask **102** allows the patient **124** to perform a plurality of activities. In addition, the patient **124** is comfortable wearing the CPAP mask 102. Further, the plurality of activities includes but may not be limited to reading books, watching Television, playing play station and sleeping in prone position.

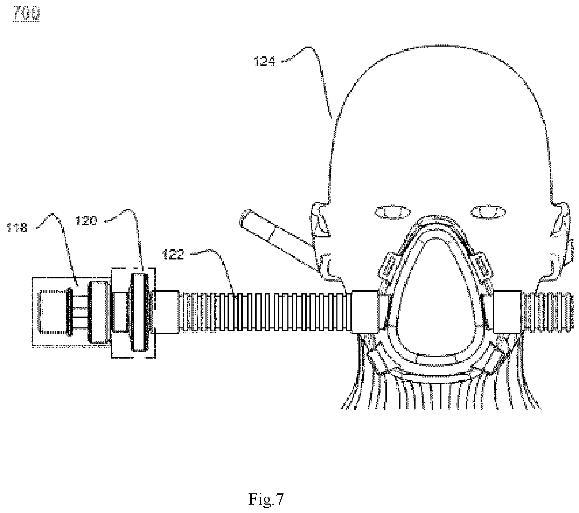

**FIG. 7** illustrates a front view **700** of a face of the patient **124** with the CPAP mask **102,** a PEEP valve **118** and a HEPA filter unit **120,** in accordance with an embodiment of the present disclosure. The front view **700** includes the patient **124,** the CPAP mask **102,** the PEEP valve **118,** the HEPA filter unit **120,** and a flexible hose **122.** In addition, the right port **108** is connected with the ventilator through the flexible hose **122.** Further, the flexible hose **122** allows continuous positive airway pressure ventilation for the patient **124.** Furthermore, the patient **124** receives the oxygenated air from the ventilator through the flexible hose **122.** In an embodiment of the present disclosure, the left port **106** is connected with the flexible hose **122.** In addition, the PEEP valve **118** and the HEPA filter unit **120** are connected to the left port **106** of the CPAP mask **102** through the flexible hose **122.** Further, the PEEP valve **118** maintains pressure of expiration air of the patient **124.** Furthermore, the HEPA filter unit **120** contains a plurality of bacteria and viruses and a plurality of particulates. Moreover, the HEPA filter unit **120** filters outflow of the expiration air of the patient **124.**

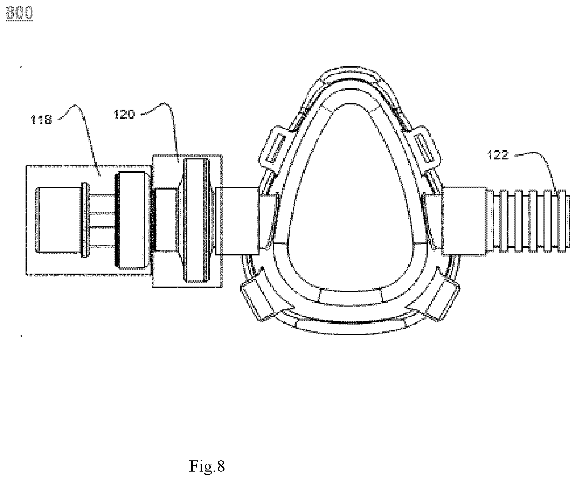

**FIG. 8** illustrates a front view **800** of the CPAP mask **102** with the PEEP valve **118** and the HEPA filter unit **120,** in accordance with another embodiment of the present disclosure. The front view **800** includes the CPAP mask **102,** the PEEP valve **118,** the HEPA filter unit **120,** and the flexible hose **122.** In addition, the right port **108** is connected with the ventilator through the flexible hose **122.** Further, the flexible hose **122** allows continuous positive airway pressure ventilation for the patient **124.** Furthermore, the patient **124** receives the oxygenated air from the ventilator through the flexible hose **122.** Moreover, the PEEP valve **118** and the HEPA filter unit **120** are connected to the left port **106** of the CPAP mask **102** directly. Also, the PEEP valve **118** maintains pressure of expiration air of the patient **124.** Also, the HEPA filter unit **120** contains the plurality of bacteria and viruses and the plurality of particulates. Also, the HEPA filter unit **120** filters outflow of the expiration air of the patient **124.**

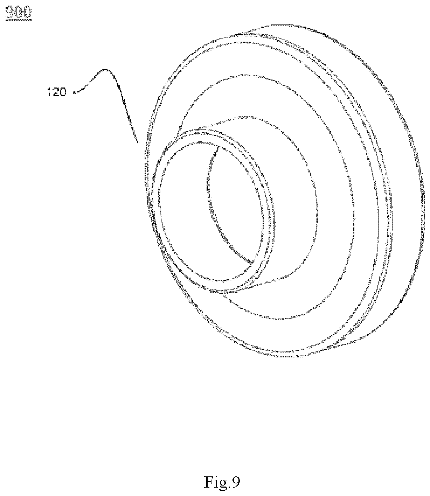

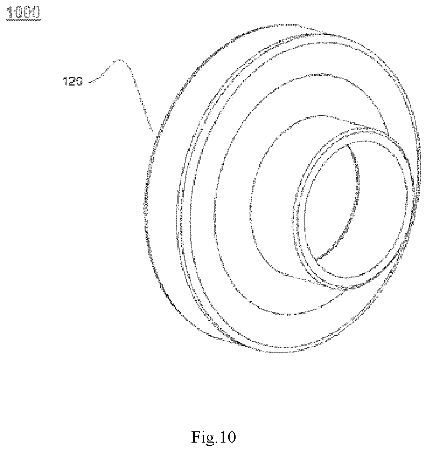

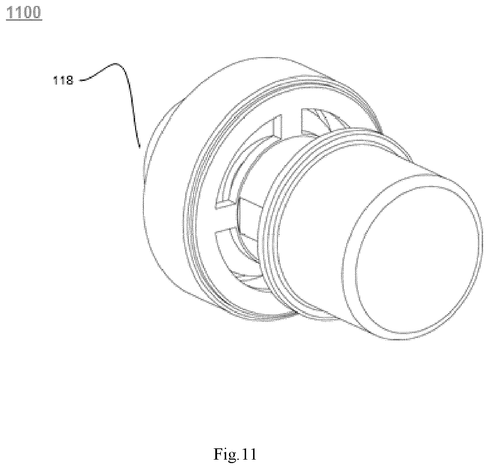

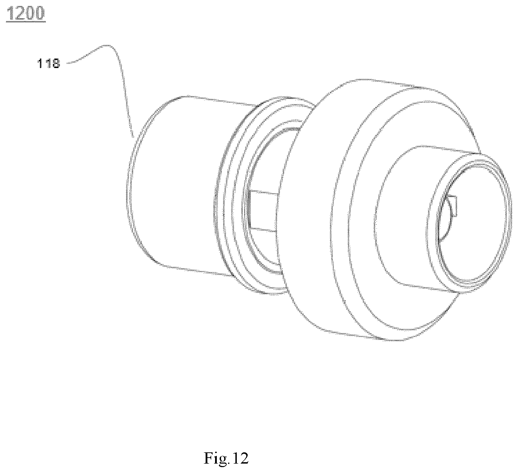

**FIG. 9** illustrates a first side view **900** of the HEPA filter unit **120,** in accordance with an embodiment of the present disclosure. **FIG. 10** illustrates a second side view **1000** of the HEPA filter unit **120,** in accordance with another embodiment of the present disclosure. **FIG. 11** illustrates a first side view **1100** of the PEEP valve **118,** in accordance with an embodiment of the present disclosure. **FIG. 12** illustrates a second side view **1200** of the PEEP valve **118,** in accordance with another embodiment of the present disclosure. The HEPA filter unit **120** corresponds to high-efficiency particulate air filter unit. In addition, the HEPA filter unit **120** includes a high energy ultra-violet light unit. Further, the high energy ultra-violet light unit eliminates the plurality of bacteria and viruses. Furthermore, the HEPA filter unit **120** provides protection against airborne disease transmission. Moreover, the HEPA filter unit **120** traps pollen and dust mites to protect the patient **124.**

The PEEP valve **118** corresponds positive end expiratory pressure valve. In addition, the PEEP valve **118** is utilized to maintain pressure on lower airways at end of breathing cycle. In an embodiment of the present disclosure, PEEP valve **118** has fixed value colour coded valves made from a transparent material that allows monitoring of respiratory rate of the patient **124.** In addition, the HEPA filter unit **120** has an inner cylindrical portion and an external cylindrical portion. Further, the PEEP valve **118** has an inner cylindrical portion and an external cylindrical portion. Furthermore, the inner cylindrical portion of the HEPA filter unit **120** connects with cylindrical portion of the left port **106.** Moreover, the external cylindrical portion of the HEPA filter unit **120** connects with the inner cylindrical portion of the PEEP valve **118.**

The present invention has various advantages over the prior art. The present invention provides a CPAP mask to provide continuous positively pressurized air to a patient. In addition, the present disclosure provides the CPAP mask to minimize pressure on face of the patient resulting in comfort. Further, the present disclosure provides the CPAP mask to allow outflow of excessive carbon dioxide exhaled by the patient. Furthermore, the present disclosure provides the CPAP mask to enable a doctor to view features of the patient through transparent front face. Moreover, the present disclosure provides the CPAP mask with side ports to facilitate treatment of the patient lying in prone position or face down position.

The foregoing descriptions of specific embodiments of the present technology have been presented for purposes of illustration and description. They are not intended to be exhaustive or to limit the present technology to the precise forms disclosed, and obviously many modifications and variations are possible in light of the above teaching. The embodiments were chosen and described in order to best explain the principles of the present technology and its practical application, to thereby enable others skilled in the art to best utilize the present technology and various embodiments with various modifications as are suited to the particular use contemplated. It is understood that various omissions and substitutions of equivalents are contemplated as circumstance may suggest or render expedient, but such are intended to cover the application or implementation without departing from the spirit or scope of the claims of the present technology.

While several possible embodiments of the invention have been described above and illustrated in some cases, it should be interpreted and understood as to have been presented only by way of illustration and example, but not by limitation. Thus, the breadth and scope of a preferred embodiment should not be limited by any of the above-described exemplary embodiments.

## Claims

1. A CPAP mask (102) with side ports for providing continuous positively pressurized air to a patient (124), the CPAP mask (102) comprising:
a mask body, wherein the mask body comprising:
a left side wall (104), wherein the left side wall (104) has a first through-hole;
a left port (106), wherein the left port (106) is on the left side wall (104);
a front face (110), wherein the front face (110) of the CPAP mask (102) is a transparent member, wherein the front face (110) allows a plurality of medical practitioners to view one or more facial features of the patient (124);
a right side wall (112), wherein the right side wall (112) has a second through-hole;
a right port (108), wherein the right port (108) is on the right side wall (112);
a plurality of strap holders (114), wherein the plurality of strap holders (114) is provided on the left side wall (104) and the right side wall (112) of the mask body, wherein the plurality of strap holders (114) clamps a medical mask strap, wherein the medical mask strap holds head of the patient (124) with the CPAP mask (102); and
a cushion seal (116), wherein the cushion seal (116) is fastened along a perimeter of the CPAP mask (102), wherein the cushion seal (116) connects the CPAP mask (102) to facial area of the patient (124).

2. The CPAP mask (102) as claimed in claim 1, wherein the CPAP mask (102) securely covers nose and mouth of the patient (124).

3. The CPAP mask (102) as claimed in claim 1 or claim 2, wherein the right port (108) is connected with a ventilator through a flexible hose (122), wherein the flexible hose (122) allows continuous positive airway pressure ventilation for the patient (124), wherein the patient (124) receives oxygenated air from the ventilator through the flexible hose (122).

4. The CPAP mask (102) as claimed in any preceding claim, wherein the left port (106) is aligned with the right port (108), wherein the alignment enables unobstructed flow of the oxygenated air from the ventilator.

5. The CPAP mask (102) as claimed in any preceding claim, wherein the cushion seal (116) flexes according to shape of the facial area of the patient (124), wherein the cushion seal (116) seals the CPAP mask (102) with the facial area of the patient (124).

6. The CPAP mask (102) as claimed in any preceding claim, wherein the left port (106) of the CPAP mask (102) is connected to a PEEP valve (118) and a HEPA filter unit (120), wherein the PEEP valve (118) maintains pressure of expiration air of the patient (124), wherein the HEPA filter unit (120) traps a plurality of bacteria and viruses and a plurality of particulates, wherein the HEPA filter unit (120) filters outflow of the expiration air of the patient (124).

7. The CPAP mask (102) as claimed in any preceding claim, wherein the CPAP mask (102) is utilized for a plurality of positions, wherein the patient (124) rests in the plurality of positions, wherein the plurality of positions comprising supine position and prone position.
